# EUROPÄISCHE PATENTANMELDUNG

(11) **EP 3 967 340 A1**
(43) Veröffentlichungstag der Anmeldung: **16.03.2022**
(21) Anmeldenummer: 20195227.2
(22) Anmeldetag: 09.09.2020
(51) Int. Cl.: A61M 5/178, A61M 5/32

(54) **MEDIZINISCHE SPRITZE MIT NADELSCHUTZ**

(71) Anmelder: Trenta2 S.r.l., 39100 Bozen (IT)
(72) Erfinder: Gallmetzer, Dietrich, 39018 Terlan (IT)
(74) Vertreter: Tergau & Walkenhorst Patentanwälte PartGmbB

(57) **Zusammenfassung**

Eine medizinische Spritze (1) mit Nadelschutz soll bei besonders einfach gehaltener und kostengünstiger Bauweise selbst unter hohen Anforderungen an die Sicherheit für den Benutzer zuverlässig vor unbeabsichtigter Verletzung oder Kontamination schützen. Dazu ist erfindungsgemäß ein den zu verabreichenden Wirkstoff enthaltender Kartuschenkörper (24), an den zur Ausbringung des Wirkstoffs ein Nadelkopf (6) anbringbar ist, außenseitig von einem in seiner Längsrichtung verschiebbaren Nadelschutzrohr (50) umgeben, das im vollständig ausgefahrenen Zustand die Hohlnadel (42) des Nadelkopfs (6) vollständig umschließt.

## Beschreibung

Die Erfindung betrifft eine medizinische Spritze mit Nadelschutz.

Zur Vermeidung oder Verringerung von Kontaminations- oder Verletzungsrisiken nach dem Gebrauch von Medikamentenspritzen und insbesondere zur Vermeidung des mehrfachen Gebrauchs von Spritzennadeln durch verschiedene Nutzer finden Spritzen mit so genannten Einzieh- oder Retraktionssystemen für die Spritzennadel zunehmend Verwendung. Bei solchen insbesondere medizinischen Spritzen, auch als "Spritze mit (passivem) Nadelschutz" bezeichnet, wird die Spritzennadel nach Abgabe des in der Spritze vorgehaltenen Wirkstoffs in den Spritzenkörper eingezogen und von diesem vollständig umschlossen. Ein Zugang zur Spritze und damit ein Verletzungsrisiko, oder auch das Risiko mehrfachen Gebrauchs derselben Nadel, kann damit weitgehend ausgeschlossen werden.

Derartige Spritzen mit passivem Nadelschutz sind beispielsweise aus der EP 1 284 769 B1, der EP 0 720 491 B1, der EP 0 680 347 B1 oder der EP 1 764 127 B1 bekannt.

Ganz allgemein werden medizinische Spritzen, insbesondere bei Spritzen für den Einweg-Gebrauch, in enormen Stückzahlen verwendet. Es besteht somit das allgemeine Bedürfnis, solche Spritzen in besonders einfach gehaltener und kostengünstiger Weise bereitzustellen.

Der Erfindung liegt nunmehr die Aufgabe zugrunde, eine Spritze mit Nadelschutz anzugeben, mit der diesem Bedürfnis selbst unter hohen Anforderungen an die Sicherheit für den Benutzer vor unbeabsichtigter Verletzung oder Kontamination Rechnung getragen ist.

Diese Aufgabe wird erfindungsgemäß gelöst mit einem den zu verabreichenden Wirkstoff enthaltenden Kartuschenkörper, an den zur Ausbringung des Wirkstoffs ein Nadelkopf anbringbar ist, und der außenseitig von einem in seiner Längsrichtung verschiebbaren Nadelschutzrohr umgeben ist, das im vollständig ausgefahrenen Zustand die Hohlnadel des Nadelkopfs vollständig umschließt.

Vorteilhafte Ausgestaltungen der Erfindung sind Gegenstand der Unteransprüche und/oder der nachfolgenden Figurenbeschreibung entnehmbar.

Die Erfindung geht von der Überlegung aus, dass die für einen besonders sicheren Umgang mit dem Spritzensystem und für eine besonders sichere Handhabung die Spritzennadel möglichst weitgehend und in möglichst vielen Situationen vollständig von einem mechanischen Schutz oder einer mechanischen Abschirmung umgeben sein sollte, um einen zufälligen Kontakt eines Benutzers mit der Nadel und damit einhergehend das Risiko einer Kontamination möglichst gering zu halten. Um dies auf besonders einfache Weise und dennoch zuverlässig zu erreichen, kann der Nadelschutz rohrförmig ausgestaltet werden, wobei das Rohr durch Verschieben in eine die Nadel vollständig umschließende Position gebracht werden kann.

Vorteilhafterweise sind der Kartuschenkörper und/oder das Nadelschutzrohr als Kunststoffteil, vorzugsweise aus Cyclo-Olefin-Polymer (COP), gefertigt. Dieser Werkstoff zeichnet sich durch hohe Bruchfestigkeit und glasähnliche Transparenz aus. Er setzt zudem keine Alkali-Ionen frei, so dass das Risiko einer pH-Wert-Verschiebung im vorgehaltenen Wirkstoff ausgeschlossen ist. Damit wird dieser Werkstoff erfindungsgemäß als für die Primärverpackung auch anspruchsvoller Medikamente, insbesondere für sensible biotechnologisch hergestellte Wirkstoffe, geeignet angesehen und verwendet. Zudem ist dieser Werkstoff für eine Fertigung im Spritzgießverfahren und somit für besonders präzise Dimensionierung geeignet.

Ein Ausführungsbeispiel der Erfindung wird anhand einer Zeichnung näher erläutert. Darin zeigen:
- Fig. 1: eine medizinische Spritze mit Nadelschutz,
- Fig. 2: die Komponenten der Spritze nach Fig. 1 in demontiertem Zustand,
- Fig. 3: die Komponenten der Spritze nach Fig. 1 im Längsschnitt,
- Fig. 4a - 4c: die Komponenten der Spritze nach Fig. 1 jeweils einzeln und vergrößert im Längsschnitt,
- Fig. 5: den Nadelkopf der Spritze nach Fig. 1 in Explosionsdarstellung, und
- Figs. 6 - 13: jeweils einige Komponenten der Spritze nach Fig. 1 zur Erläuterung der Vorgehensweise bei der Benutzung.

Gleiche Teile sind in allen Figuren mit denselben Bezugszeichen versehen.

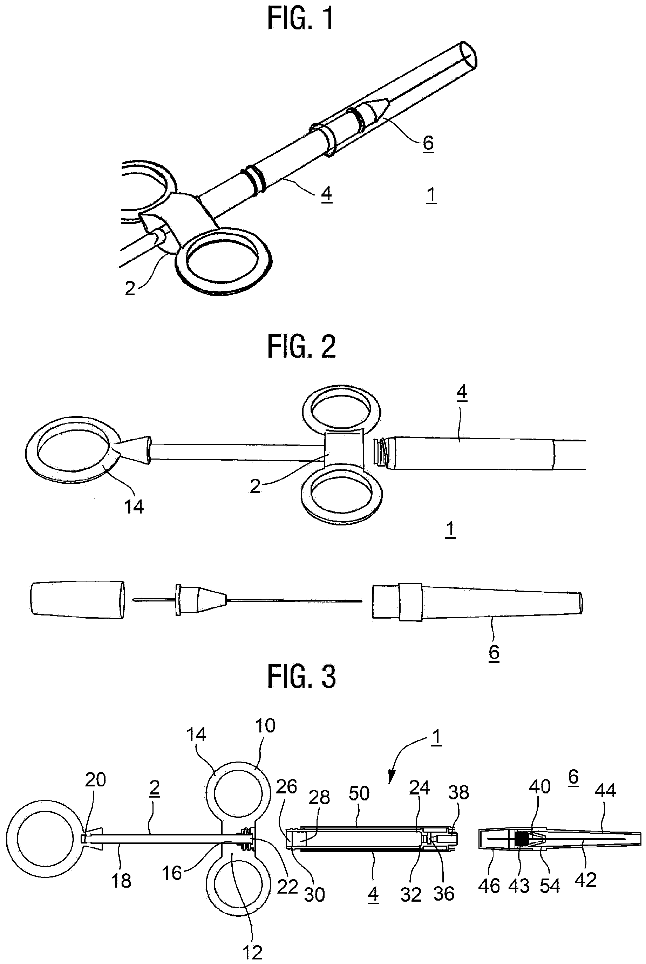

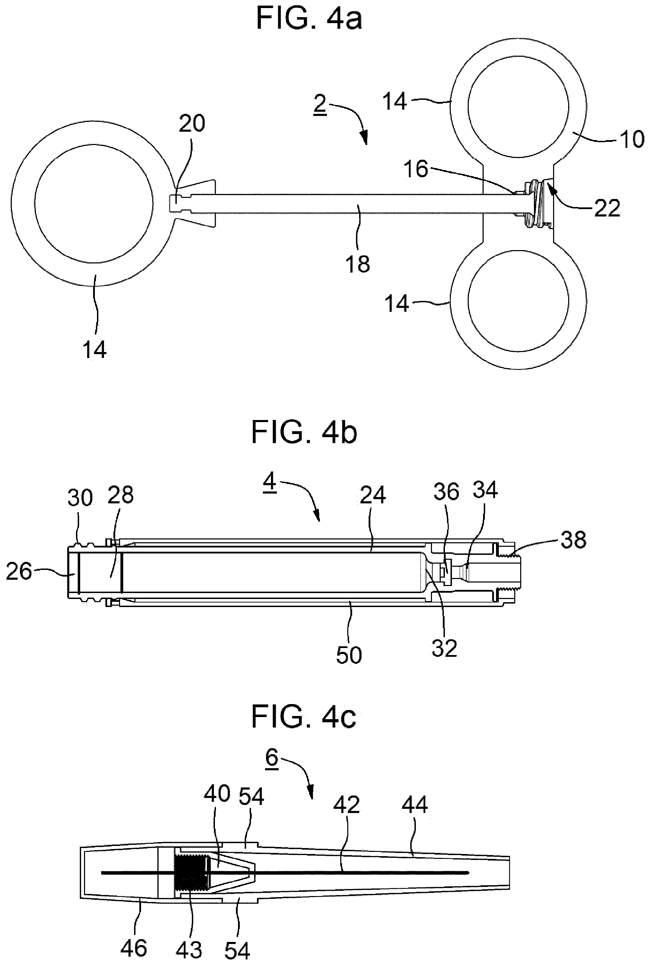

Die medizinische Spritze 1 mit Nadelschutz, wie sie in ihrer Gesamtheit in den Figuren 1,2 und 3 gezeigt ist, umfasst im Wesentlichen drei Grundkomponenten, nämlich ein Griffstück 2 (vergrößert dargestellt in Fig. 4a), eine Kartuschen- oder Patroneneinheit 4 (vergrößert dargestellt in Fig. 4b) und einen Nadelkopf 6 (vergrößert dargestellt in Fig. 4c). Das Griffstück 2 ist dabei als Mehrwegprodukt ausgeführt und zum wiederholten Einsatz vorgesehen. Demgegenüber sind die Kartuschen- oder Patroneneinheit 4 und der Nadelkopf 6 jeweils als Einweg- oder Wegwerfprodukt ausgeführt und zum lediglich einmaligen Gebrauch vorgesehen. Der Nadelkopf 6 ist auf die Kartuschen- oder Patroneneinheit 4 auf- und diese wiederum an das Griffstück 2 anschraubbar.

Die medizinische Spritze 1 ist konsequent für eine besonders hohe Bedienungssicherheit bei einfach gehaltener Bauweise ausgelegt. Durch die Bauweise soll insbesondere, zum Schutz des medizinischen Personals, die Gefahr ausgeräumt oder zumindest minimiert werden, dass sich der Bediener mit der Nadel der Spritze stechen und damit einer Kontamination ausgesetzt sein könnte. Des Weiteren soll aber auch eine besonders hohe Zuverlässigkeit bei der Handhabung der medizinischen Wirkstoffe gewährleistet sein. Im Hinblick auf diese Vorgaben sind unter anderem auch die Materialien der Komponenten der Spritze 1 geeignet gewählt.

Das Griffstück 2 umfasst dabei einen Halterahmen 10 mit einem Grundkörper 12, an den zwei im Ausführungsbeispiel als Eingriffsringe 14 ausgeführte Fingerstücke angeformt sind. Der Grundkörper 12 weist in der Art einer zentralen Bohrung einen durchgehenden Führungskanal 16 auf, in dem ein Betätigungsstößel 18 verschiebbar gelagert ist. Am freien oder proximalen Ende 20 des Betätigungsstößels 18 ist ein weiteres, ebenfalls als Eingriffsring 14 ausgeführtes Fingerstück angeordnet. Der Führungskanal 16 weitet sich ausgangsseitig auf und ist in seinem Endbereich mit einem Innengewinde 22 versehen, in das ein entsprechendes Außengewinde der Kartuschen- oder Patroneneinheit 4 eingeschraubt werden kann. Diese Gewindeverbindung kann insbesondere als an sich übliches Luer-Gewinde ausgeführt sein.

Im Hinblick auf die genannten Auslegungsziele einer an sich einfach gehaltenen, aber hochwertigen und insbesondere hohen Sicherheitserfordernissen genügenden Bauweise ist der Halterahmen 10, ebenso wie das weitere Fingerstück, aus Polyphenylsulfon (PPSU) ausgeführt, einem hochtechnischen Spezialkunststoff, der bei hoher mechanischer Stabilität und Belastbarkeit nahezu beliebig oft sterilisierbar ist und bei medizinischen Produkten als Metallersatz gilt. Der Betätigungsstößel 18 ist hingegen aus Chromstahl ausgeführt. In seiner Gesamtheit ist das Griffstück 2 somit, insbesondere aufgrund seiner Materialwahl, vielfach und wiederholt sterilisierbar und somit vielfach wiederverwendbar.

Die gezeigte Kartuschen- oder Patroneneinheit 4 der Spritze 1 ist in der Art einer Ampulle mit dem zu verabreichenden medizinischen Wirkstoff vorbefüllt. Sie umfasst den eigentlichen, als zylindrischen Hohlkörper ausgestalteten Kartuschenkörper 24, der an seinem proximalen Ende 26 mit einem im Kartuschenkörper 24 verschiebbaren Kolben 28 verschlossen ist. Der Kartuschenkörper 24 ist an seinem proximalen Ende 26 zudem mit einem von seinen Abmessungen her an das Innengewinde 22 des Griffstücks 2 angepassten Außengewinde 30 versehen, so dass der Kartuschenkörper 24 an das Griffstück 2 angeschraubt werden kann. An seinem distalen Ende 32 mündet der Kartuschenkörper 24 hingegen in einen angeformten Ausströmkanal 34, der mit einer Membran 36 verschlossen und in seinem Endbereich außenseitig mit einem Außengewinde 38 versehen ist. In dem in den Figuren 2, 3 und 4b gezeigten, noch nicht fertig montierten Zustand der Spritze 1 wird der den medizinischen Wirkstoff enthaltende Innenraum des Kartuschenkörpers 24 somit vom Kolben 28 einerseits und der Membran 36 andererseits dicht verschlossen, so dass der Wirkstoff darin sicher gelagert und vorgehalten werden kann.

Die Kartuschen- oder Patroneneinheit 4 ist als Einwegprodukt ausgelegt und für einen lediglich einmaligen Gebrauch vorgesehen. Die Materialwahl ist dabei insbesondere im Hinblick auf die zuverlässige vorübergehende Lagerung des medizinischen Wirkstoffs einhergehend mit einer besonders hohen Sicherheit im Umgang mit den Komponenten getroffen. Der als zylindrischer Hohlkörper ausgeführte Kartuschenkörper 24 ist dabei aus dem Hochleistungskunststoff Cyclo-Olefin-Polymer gefertigt. Dieser Werkstoff zeichnet sich durch hohe Bruchfestigkeit und glasähnliche Transparenz aus. Er setzt zudem keine Alkali-Ionen frei, so dass das Risiko einer pH-Wert-Verschiebung im vorgehaltenen Wirkstoff ausgeschlossen ist. Der Kartuschenkörper 24 ist vorzugsweise im Spritzgießverfahren gefertigt, wobei unter anderem die Formgebung derart erfolgt, dass mögliche Totvolumina im Inneren besonders gering gehalten sind. Der den Stopfen der Spritze 1 bildende Kolben 28 ist hingegen aus thermoplastischem Polymer (TPE) gefertigt, wobei der Kartuschenkörper 24 und der darin geführte Polymerstopfen vorzugsweise im zwei-Komponenten-Spritzgießverfahren in lediglich einem Fertigungsschritt gefertigt werden.

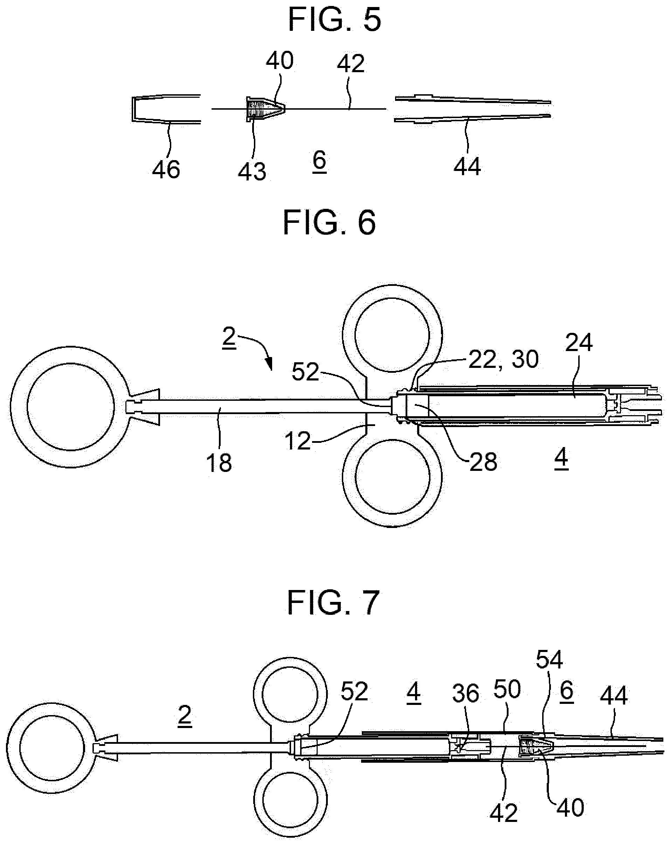

Der in Fig. 3 und Fig. 4c im Längsschnitt und in Fig. 5 in Explosionsdarstellung gezeigte Nadelkopf 6 umfasst den Nadelhalter 40, in dem zentral eine im Ausführungsbeispiel als Standardnadel 27 G 0.4x38mm ausgeführte Hohlnadel 42 geführt ist, und der innenseitig mit einem an das Außengewinde 38 des Kartuschenkörpers 24 angepassten Innengewinde 43 ausgerüstet ist. Damit ist der Nadelhalter 40 auf den Kartuschenkörper 24 aufschraubbar. Im in den Figuren 3, 4c und 5 gezeigten Zustand vor der endgültigen Montage der Spritze 1 umfasst der Nadelkopf 6 zudem eine erste, distale Nadelschutzkappe 44 und eine zweite, proximale Nadelschutzkappe 46. Diese sind im in Fig. 3 und 4c gezeigten Zustand vor der Montage ineinandergesteckt und umschließen die Hohlnadel 42 vollständig und allseitig, so dass sich der Benutzer nicht versehentlich stechen kann.

Die aus den beschriebenen Komponenten gebildete medizinische Spritze 1 ist zudem im Hinblick auf den angestrebten Schutz des Bedieners oder medizinischen Personals gegen eine Kontamination oder Infektion, insbesondere durch versehentliches Stechen mit der benutzten Nadel, mit einem aktiven Nadelschutz versehen. Damit wird bezweckt, dass nach der Benutzung der Spritze 1, also nach der Ausgabe des im das Spritzengehäuse bildenden Kartuschenkörper 24 vorgehaltenen Wirkstoffs über die Nadel 42, diese auf geeignete Weise vollständig umschlossen wird. Damit soll eine unbeabsichtigte Berührung der benutzten Nadel 42, beispielsweise durch Hilfs- oder Pflegepersonal, und somit das Verletzungs-und Kontaminationsrisiko besonders geringgehalten oder möglichst ganz ausgeschlossen werden.

Um dies zu gewährleisten, ist der Kartuschenkörper 24 von einem auf diesem verschiebbar gelagerten Nadelschutzrohr 50 umgeben. Um die angestrebte hohe Sicherheit bei der Handhabung zu gewährleisten, ist grundsätzlich der folgende Ablauf beim Einsatz und bei der Handhabung der genannten Komponenten vorgesehen:
In einem ersten Schritt bei der Benutzung wird, wie in Fig. 6 dargestellt, die Kartuschen- oder Patroneneinheit 4 bzw. die Ampulle mit dem Griffstück 2 verbunden, indem das Außengewinde 30 des Kartuschenkörpers 24 in das Innengewinde 22 im Grundkörper 12 des Griffstücks 2 eingeschraubt wird. Damit kann die freie Stirnfläche 52 des Betätigungsstößels 18 an den Kolben 28 im Kartuschenkörper 24 angelegt werden.

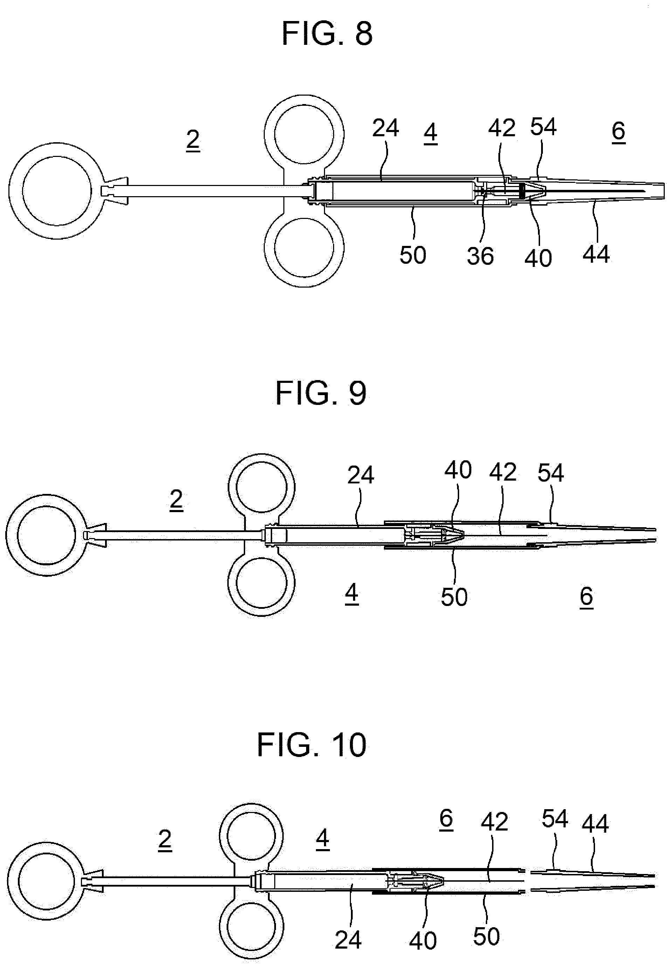

Im nächsten Schritt wird, wie in den Figuren 7 und 8 gezeigt, der Nadelkopf 6 an der Kartuschen- oder Patroneneinheit 4 angebracht. Dazu wird zunächst die proximale Nadelschutzkappe 46 vom Nadelhalter 40 abgezogen, so dass das proximale Ende der Hohlnadel 42 freigelegt wird. Anschließend wird, wie in Fig. 7 gezeigt, das den Kartuschenkörper 24 umgebende Nadelschutzrohr 50 etwas zum freien Ende hin verschoben, so dass der - in seinen Abmessungen entsprechend an das Nadelschutzrohr 50 angepasste - untere Teil des Nadelhalters 40 in das freie Ende des Nadelschutzrohrs 50 eingesteckt werden kann und dort geführt wird. Zur Erleichterung der Positionierung ist die sich in diesem Moment immer noch am Nadelhalter 40 befindende distale Nadelschutzkappe 44 mit einer umlaufenden Wulst 54 versehen, die einen Anschlag für das freie Ende des Nadelschutzrohrs 50 bildet. Anschließend kann der solchermaßen in das Nadelschutzrohr 50 eingesteckte Nadelhalter 40 durch Zurückschieben des Nadelschutzrohrs 50 bzw. Aufschrauben des Innengewindes 43 des Nadelhalters 40 auf das Außengewinde 38 am Kartuschenkörper 24 in geführter Bewegung mit dem Kartuschenkörper 24 in Kontakt gebracht werden, bis die Nadel 42 die Membran 36 im Kartuschenkörper 24 durchstößt. Wie im in Fig. 8 gezeigten Zustand ist somit anschließend das Innengewinde 43 im Nadelhalter 40 auf das den Ausströmkanal 34 umgebende Außengewinde 38 aufgeschraubt, so dass der Nadelhalter 40 sicher mit dem Kartuschenkörper 24 verbunden ist. Die distale Nadelschutzkappe 44 verbleibt dabei unverändert auf dem Nadelhalter 40, so dass keine Verletzungsgefahr für den Benutzer besteht.

Anschließend wird ausgehend von diesem in Fig. 8, 9 gezeigten Zustand die distale Nadelschutzkappe 44 entfernt, indem das Nadelschutzrohr 50 wieder nach außen hin verschoben wird. Durch den von der Wulst 54 gebildeten Anschlag wird bei dieser Bewegung des Nadelschutzrohrs 50 die Nadelschutzkappe 44 mitgenommen und letztlich abgestreift, wobei der Benutzer lediglich das Nadelschutzrohr 50 berühren muss. Wie in Fig. 10 gezeigt, ist das Nadelschutzrohr 50 nach diesem Vorgang vollständig ausgefahren und umgibt - aufgrund seiner geeignet gewählten Dimensionierung und Länge - die Nadel 42 vollständig. Auch nach dem Entfernen der distalen Nadelschutzkappe 44 ist somit die Nadel 42 vollständig umschlossen und nicht zugänglich; ein Verletzungsrisiko ist somit auch hier ausgeschlossen.

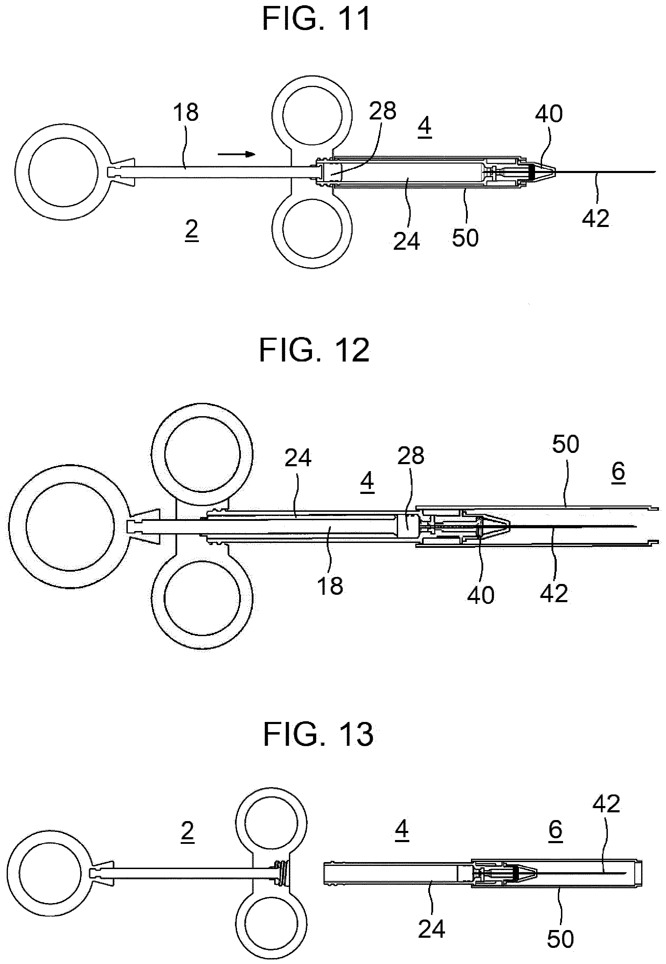

Zur Verabreichung des Wirkstoffs kann anschließend, wie dies in Fig. 11 gezeigt ist, das Nadelschutzrohr 50 wieder zurückgezogen werden, so dass das distale Ende der Hohlnadel 42 nunmehr freiliegt. Die Spritze 1 ist somit zur Verabreichung des Wirkstoffs einsatzbereit. Wie in Fig. 11 angedeutet, wird dann zur Verabreichung des Wirkstoffs der Betätigungsstößel 18 in Richtung zur Hohlnadel 42 hin verschoben, wobei der Kolben 28 innerhalb des Kartuschenkörpers 24 verschoben wird und damit den Wirkstoff über die Hohlnadel 42 ausbringt.

Nach Verabreichung des Wirkstoffs wird dann, wie in Fig. 12 gezeigt, das Nadelschutzrohr 50 wieder vollständig ausgefahren, so dass es erneut die Hohlnadel 42 vollständig umschließt und der Benutzer somit erneut vor Verletzungen oder unbeabsichtigtem Stechen geschützt ist. Anschließend kann dann, wie in Fig. 13 gezeigt, das Ensemble aus Kartuschen- oder Patroneneinheit 4 und Nadelkopf 6 vom Griffstück 2 abgeschraubt und der Entsorgung zugeführt werden. Auch dabei verbleibt das Nadelschutzrohr 50 in der vollständig ausgefahrenen Position, so dass auch bei den nachfolgenden Handhabungen, z. B. bei der Entsorgung, die beteilgten Personen vor unabsichtlichem Stechen geschützt sind.

### Bezugszeichenliste

- 1: Medizinische Spritze
- 2: Griffstück
- 4: Kartuschen- oder Patroneneinheit
- 6: Nadelkopf
- 10: Halterahmen
- 12: Grundkörper
- 14: Eingriffsring
- 16: Führungskanal
- 18: Betätigungsstößel
- 20: proximales Ende
- 22: Innengewinde
- 24: Kartuschenkörper
- 26: proximales Ende
- 28: Kolben
- 30: Außengewinde
- 32: distales Ende
- 34: Ausströmkanal
- 36: Membran
- 38: Außengewinde
- 40: Nadelhalter
- 42: Hohlnadel
- 43: Innengewinde
- 44: distale Nadelschutzkappe
- 46: proximale Nadelschutzkappe
- 50: Nadelschutzrohr
- 52: Stirnfläche
- 54: Wulst

## Patentansprüche

1. Spritze (1) mit Nadelschutz, mit einem den zu verabreichenden Wirkstoff enthaltenden Kartuschenkörper (24), an den zur Ausbringung des Wirkstoffs ein Nadelkopf (6) anbringbar ist, und der außenseitig von einem in seiner Längsrichtung verschiebbaren Nadelschutzrohr (50) umgeben ist, das im vollständig ausgefahrenen Zustand die Hohlnadel (42) des Nadelkopfs (6) vollständig umschließt.

2. Spritze (1) nach Anspruch 1, deren Kartuschenkörper (24) als Kunststoffteil, vorzugsweise aus Cyclo-Olefin-Polymer (COP), gefertigt ist.

3. Spritze (1) nach Anspruch 1 oder 2, deren Nadelschutzrohr (50) als Kunststoffteil, vorzugsweise aus Cyclo-Olefin-Polymer (COP), gefertigt ist.
